# EUROPEAN PATENT APPLICATION

(11) **EP 4 102 210 A1**
(43) Date of publication of application: **14.12.2022**
(21) Application number: 21386033.1
(22) Date of filing: 07.06.2021
(51) Int. Cl.: G01N 21/64, G01N 21/552, B01L 3/00, B82Y 15/00, C12Q 1/6869, G01N 33/487, G02B 6/30, G02B 6/122, G11C 13/04, G11C 11/42, G01N 21/65

(54) **NANOPHOTONIC NANOPORE, MEMORY SYSTEM COMPRISING A NANOPHOTONIC NANOPORE, AND METHOD OF READING AND/OR WRITING INFORMATION FROM AND/OR TO AN ANALYTE**

(71) Applicant: Oxford University Innovation Limited, Botley Oxford OX2 0JB (GB)
(72) Inventor: BHASKARAN, Harish, Oxford (GB); MOL, Jan, Oxford (GB); FARMAKIDIS, Nikolaos, Oxford (GB); SWETT, Jacob, Oxford (GB); YOUNGBLOOD, Nathan, Oxford (GB)
(74) Representative: Barker Brettell LLP

(57) **Abstract**

A nanophotonic nanopore (100) is provided, comprising a nanopore (110) and an optical waveguide (105). The optical waveguide (105) is optically coupled to the nanopore (110) so that a transmission, reflection, emission or absorption property of the optical waveguide (105) is modified by an analyte received by the nanopore (110).

## Description

### FIELD OF THE INVENTION

The invention relates to a nanopore, and more specifically to a nanopore with optical transduction. The invention also relates to a system for reading and/or writing data.

### BACKGROUND

Nanopores can be used to analyse molecules, for example to sequence a DNA molecule. A nanopore is typically disposed in a membrane and a voltage is used to drive charged particles through the nanopore (by electrophoresis). Molecules transiting through the nanopore will alter conduction properties between electrodes disposed around the pore. The characteristics of the region of the molecule that is transitioning through the pore will affect the conduction properties. Changes in current density (for example) can be used to characterise base pairs as they transit through the nanopore. This technique may be used for rapid sequencing of DNA: Oxford Nanopore Technologies is an example of a company working to develop this type of technology.

Optical readout of the local characteristics of molecules in the region of a nanopore has been proposed¹, via wide field total internal reflection microscopy via the membrane (in which the nanopore is defined) to detect fluorescence.
¹ Soni, Gautam V., et al. "Synchronous optical and electrical detection of biomolecules traversing through solid-state nanopores." Review of Scientific Instruments 81.1 (2010): 014301.

Improved readout and interaction mechanisms for nanopores are desirable.

One example of an application for such improved readout and interaction mechanisms is in data reading and writing. Encoding data in the local characteristics of molecules/polymers (for example, as a "molecular tape", for example as in base pairs of DNA²) has the promise of providing exceptional storage density and long lifetime. Nanopores may be an enabling technology for molecular/polymer-based data reading and/or writing.
² Chen, Kaikai, et al. "Digital data storage using DNA nanostructures and solid-state nanopores." Nano letters 19.2 (2018): 1210-1215.

### SUMMARY

According to a first aspect, there is provided a nanophotonic nanopore comprising:
a nanopore, and an optical waveguide;
wherein the optical waveguide is optically coupled to the nanopore so that a transmission, reflection, emission or absorption property of the optical waveguide is modified by an analyte received by the nanopore.

The nanopore may be less than 200nm in diameter, or less than 100nm in diameter, or less than 50nm in diameter.

The provision of the waveguide enables a more scalable solution, in which arrays of devices are provided. The waveguide may be used to produce a very highly concentrated light field at the nanopore, enabling more precise characterisation of the analyte. The waveguide (and optionally any arrangement for localising/intensifying the field at the nanopore) may enable sufficient field intensity to enable changing the properties of the analyte in the region of the nanopore, to write information as well as read.

The optical waveguide may comprise a first waveguide portion and a second waveguide portion that are coupled together via the nanopore.

The nanophotonic nanopore may further comprise a field enhancement structure (e.g. a plasmonic enhancement structure) configured to enhance an electrical field strength at the nanopore by field confinement of an optical signal carried by the optical waveguide. Plasmonic enhancement may be used to effectively focus an optical field to a very small volume corresponding with the position of the nanopore, so as to effect very sensitive localised interrogation of the characteristics of a region of an analyte captured by the nanopore (and optionally localised changing of the characteristics of the region to effect writing as well as reading characteristics).

The plasmonic enhancement structure may comprise a first region and second region, spaced apart by a nanogap at the nanopore. The nanogap may be the position in which the field is enhanced by the plasmonic enhancement structure.

The nanogap may be 200nm or less. The nanogap may be a small fraction (e.g. less than 1/2 or less than 1/5) of the wavelength of the light used to interrogate the nanopore. The use of a field enhancement structure (e.g. plasmonic) enables very small regions of analytes to be characterised with precision using light, removing the diffraction limit.

The first waveguide portion may comprise a narrowing taper at an end of the first waveguide portion adjacent to the nanopore. The second waveguide may comprise a narrowing taper at an end of the second waveguide portion adjacent to the nanopore. The plasmonic enhancement structure may be spaced apart from the narrowing tapers of the first and second waveguide portions by a taper nanogap.

The taper nanogap may be 200nm or less (or 100nm or less, or 50nm or less).

The plasmonic enhancement structure may comprise a material capable of supporting surface plasmon polaritons.

The plasmonic enhancement structure may comprise: silver, gold, aluminium, copper, titanium nitride, a heavily doped semiconductor material, or a 2D material (e.g. one or less than 5 atomic layers thick).

The nanophotonic nanopore may further comprise a first electrode and a second electrode, separated by a plasmonic nanogap at the nanopore.

The optical waveguide may be configured as a photonic crystal comprising at least one cavity, and the nanopore may be disposed within one of the at least one cavities.

The photonic crystal may be configured to enhance the electric field in the region of the nanopore resulting from an optical signal propagating through the optical waveguide (e.g. the photonic crystal may provide a field enhancement structure instead of a plasmonic enhancement structure).

The optical waveguide may comprise one of a plurality of optical waveguides in a stacked configuration, each of the optical waveguides optically coupled to the nanopore so that a transmission property of each optical waveguide is modified by a respective region of the analyte at the position of that optical waveguide.

The optical waveguide may comprise a rib waveguide.

The waveguide may be configured to carry light with a wavelength that is at least twice a diameter of the nanopore.

According to a second aspect, there is provided a memory system, comprising:
a nanophotonic nanopore according to the first aspect, and
an analyte comprising one or more information encoding regions that are configured to change a characteristic in response to an optical signal carried by the optical waveguide when the information encoding region is within the nanopore;
wherein the nanophotonic nanopore is configured to write information to the analyte by changing the characteristic of the information encoding region.

According to a third aspect, there is provided a memory system, comprising:
a nanophotonic nanopore according to the first aspect, and
an analyte comprising one or more information encoding regions that comprise a characteristic that can be inferred by transmission properties of the optical waveguide when the information encoding region is within the nanopore;
wherein the nanophotonic nanopore is configured to read information encoded in the characteristic of the information encoding regions of the analyte.

The first and second aspect may be combined (i.e. the memory system may be configured to both read and write).

The analyte may comprise a polymer.

The encoding region may comprise a fluorophore.

The encoding region may comprise a first fluorophore and a second fluorophore, and the first and second fluorophore may be coupled by fluorescence resonance energy transfer, so that excitation of the first fluorophore results in emission from the second fluorophore.

The memory system is configured so that writing to the encoding region comprises bleaching the encoding region to reduce a fluorescence resonance energy transfer. The characteristic may comprise a degree of fluorescence resonance energy transfer.

The encoding region may be configured to encode more than one bit of information.

The nanophotonic nanopore may be configured to read multiple bits of information in parallel using different wavelengths.

According to a fourth aspect, there is provided a method of reading and/or writing information from an analyte, comprising:
capturing the analyte in a nanophotonic nanopore, the nanophotonic nanopore comprising a nanopore and an optical waveguide optically coupled to the nanopore;
optically reading and/or writing information from/to the analyte using an optical signal carried by the waveguide.

The method may comprise using the nanophotonic nanopore of the first aspect or the memory system of the second or third aspect.

The features of each aspect, including the optional features thereof, may be combined with those of each other aspect. Features described with reference to the embodiments may likewise be combined with the aspects and optional features disclosed above.

### BRIEF DESCRIPTION OF DRAWINGS

Embodiments of the invention will be described, by way of example only, with reference to the accompanying drawings, in which:
Figure 1 is a schematic diagram of a nanophotonic nanopore, comprising a tapering plasmonic enhancement structure;
Figures 2 and 3 show plan view simulations of field distribution at a nanopore as a result of plasmonic enhancement with and without an analyte particle present;
Figure 4 shows a section view of simulated field distribution in the region of the nanopore with no particle, and with a 25nm gold particle at various positions in the z direction;
Figure 5 is a cross section through the nanophotonic nanopore of Figure 1 along AA;
Figure 6 is a cross section through the nanophotonic nanopore of Figure 1 along BB showing fluid input and output;
Figure 7 is an alternative cross section through the nanophotonic nanopore of Figure 1 along BB showing fluid input and output;
Figure 8 is a micrograph of an example nanophotonic nanopore;
Figure 9 is a micrograph of an example nanophotonic nanopore with a droplet over the nanopore;
Figure 10a and 10b are micrographs illustrating a fabricated tapering plasmonic enhancement structure (prior to and after forming the nanopore);
Figures 11 to 14 are graphs showing transmission properties of the waveguide varying with particle trapping at the nanopore;
Figure 15 is a rendering of an example system comprising a nanophotonic nanopore;
Figure 16 is a schematic diagram of a nanophotonic nanopore, comprising a plasmonic enhancement structure including plasmonic nano-discs;
Figure 17 is a schematic diagram of a nanophotonic nanopore, comprising a plasmonic antenna;
Figure 18 is a schematic diagram of a nanophotonic nanopore, comprising a photonic crystal cavity;
Figure 19 shows a device comprising a plurality of nanopores, each coupled to a different ring resonator, for example to enable wavelength division multiplexed readout of each nanopore;
Figure 20 shows a nanophotonic nanopore in which the nanopore is coupled to a ring resonator and comprising a tapering plasmonic enhancement structure;
Figure 21 shows a unit of an example information storing polymer, comprising a pair of fluorescent proteins that interact via fluorescence resonance energy transfer (FRET);
Figure 22 is a schematic of an information storing polymer being optically read/written by a nanophotonic nanopore;
Figure 23 is a schematic of an information storing polymer with subunits comprising a plurality of functional groups, each interrogated (i.e. read/write) by a different wavelength; and
Figure 24 is a schematic of a stacked arrangement of nanophotonic nanopores, configured to interrogate (read/write) different subunits of an information storing polymer;

### DETAILED DESCRIPTION

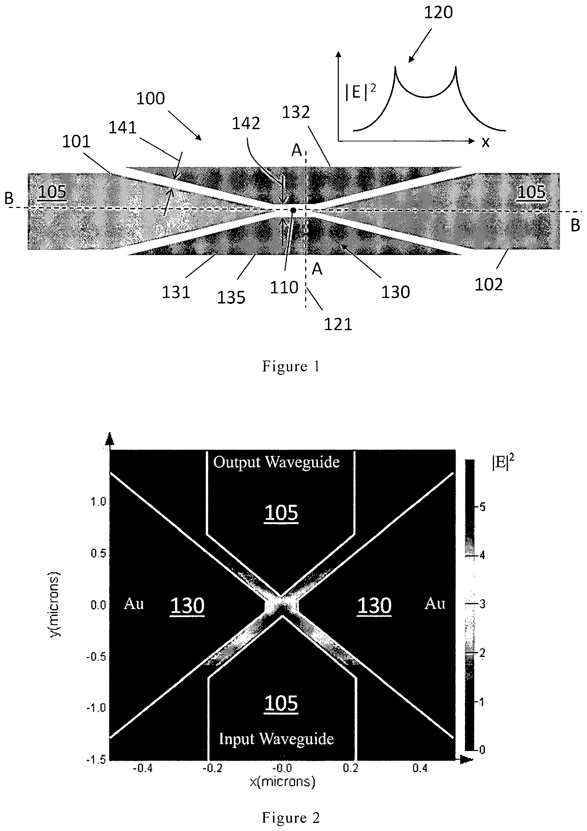

Figure 1 shows a nanophotonic nanopore 100, comprising: waveguide 105, nanopore 110, and plasmonic enhancement structure 130. The waveguide 105 comprises a first waveguide portion 101 and a second waveguide portion 102. The nanopore 110 is disposed between the first waveguide portion 101 and the second waveguide portion 102, so that light is coupled from the first waveguide portion 101 to the second waveguide portion 102 via the nanopore 110. Each of the first and second waveguide portions 101, 102 tapers to a point proximate to the nanopore 110 (i.e. a male taper).

The plasmonic enhancement structure 130 comprises a bowtie type structure, having a first and second female taper that respectively receive the male tapers of the first and second waveguide portions 101, 102. The first and second female taper are separated from the respective male tapers of the waveguide by a taper gap 141, which may be substantially constant (i.e. the sloped portions of the male and female tapers may be at the same angle to the direction of light propagation through the waveguide 105). It is not essential that the tapers have matching angles - in some embodiments the gap between the male and female taper may vary. The plasmonic enhancement structure 130 comprises a first region 131 and second region 131, separated by a plasmonic nanogap 142 at the nanopore 110. The plasmonic nanogap 142 may be 200nm or less, for example 50nm.

The plasmonic enhancement structure 130 is configured to cause plasmonic enhancement of light carried by the optical waveguide 105 at the nanopore 110. This is illustrated in Figure 1 by the inset graph 120, which schematically illustrates the magnitude of the electric field along line 121 resulting from light propagating through the waveguide 105. There is significant enhancement of the electric field in the region of the nanopore 110. Examples of simulations illustrating field enhancement are shown in Figures 2 to 4.

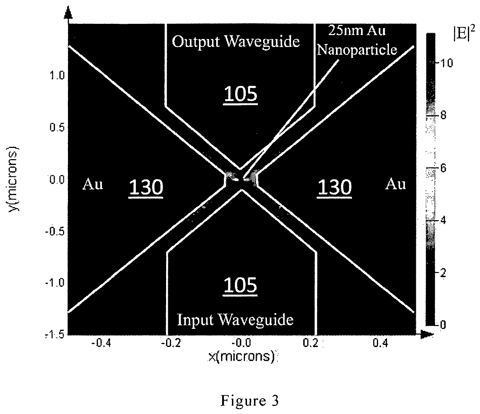

Figure 2 shows a (plan view) simulation of electric field at an example plasmonic enhancement structure 130, resulting from light propagating in the waveguide 105, with no particle present. It is clear that there is considerable enhancement of the electric field in the region of the gap, as a result of the interaction of the field with the plasmonic enhancement structure 130. Figure 3 shows a simulation corresponding with that of Figure 2, but in which a particle is present in the nanopore (in this case a 25nm gold nanoparticle). The gold nanoparticle causes a considerable changein the distribution of the electric field, which will result in a measurable change in the transmission (reflection or absorption) properties of the waveguide 105. It can be expected that a fluorescent particle may change the emission properties of the waveguide 105, by causing light to be re-emitted at a different waveglength.

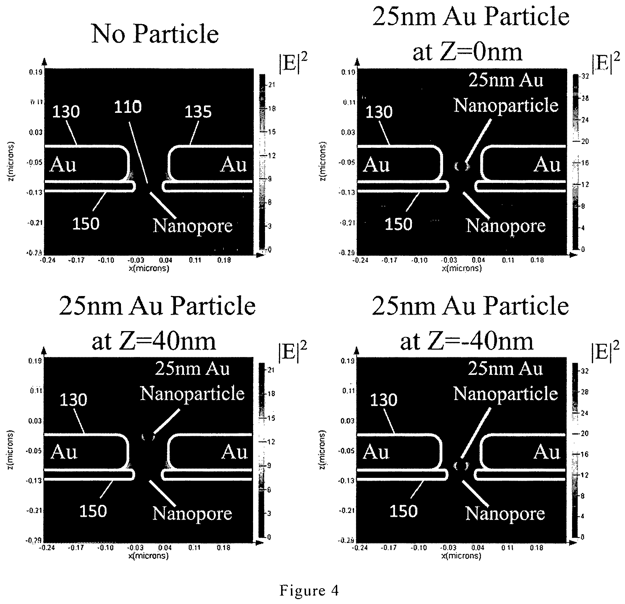

Figure 4 shows four simulations of electric field distribution through the depth of the nanopore through a section of an example nanopore, showing electric field distributions with i) no particle present; ii) a 25nm gold particle at the mean thickness position of the plasmonic enhancement structure 130; iii) a 25nm gold particle at +40nm from the mean thickness position (i.e. at the top of the plasmonic enhancement structure 130); and iv) a 25nm gold particle at -40nm from the mean thickness position (i.e. at the botton of the plasmonic enhancement structure 130). The maximum perturbation of the electrical field distribution occurs when the particle is at the mean thickness of the plasmonic enhancement structure. Although in this example the thickness of the plasmonic enhancement structure is greater that the analyte, this is not essential, and optical interrogation of regions of larger analytes are envisaged in certain embodiments (which will be discussed in more detail below).

In this example, the plasmonic enhancement of the electric field in the region of the nanopore 110 results in significant (i.e. measurable) changes in the transmission, reflection or absorption properties of the waveguide 105 resulting from the local characteristics of an analyte in the nanopore 110. For example, the waveguide 105 may be used to identify one or more characteristic of a region of the analyte that is within the pore 110 (e.g. from a spectrum, or some other optical property). A spectrum may be used to characterise the region of the analyte, for example to determine a property that encodes information (e.g. base pair identification for DNA).

Although plasmonic enhancement is an elegant way to achieve optical coupling between the waveguide 105 and the nanopore 110, it is not essential, and other embodiments may employ different mechanisms for field enhancement and localisation in the region of the nanopore 110.

The plasmonic enhancement structure 130 may comprise any suitable material (capable of supporting plasmonic enhancement of light carried by the waveguide 105 in the region of the nanopore 110). Examples of suitable materials include: gold, silver, aluminium, copper, titanium nitride and heavily doped semiconductors (such as zinc oxide, indium oxide, indium tin oxide etc.). The thickness of the material comprising the plasmonic enhancement structure 130 may be less than 200nm (e.g. 75nm). The thickness of the plasmonic enhancement sutrcture 130 may be in the range 20nm to 100nm.

In addition to providing plasmonic enhancement, the plasmonic enhancement structure 130 in the example of Figure 1 forms a pair of electrodes on either side of the nanopore 110. The electrodes may be used to electrically interrogate the nanopore 110 to determine one or more characteristic of a region of an analyte within the pore that is adjacent the plasmonic enhancement structure. For example, tunnelling current may be used to identify particular characteristics (e.g. base pairs, or other characteristics that can encode information) of an analyte within the nanopore 110.

The nanopore 110 may be less than 200nm in diameter (or less than 100nm in diameter), for example 50nm. In some embodiments the nanopore 110 may be less than 20nm in diameter. The nanopore 110 may comprise a "solid state" pore, in the sense that the membrane within which the nanopore 110 is defined may be lipid-free (and/or inorganic). For example, the nanopore 110 may be formed in a silicon nitride layer (which is compatible with typical semiconductor processing e.g. CMOS). In some embodiments, the nanopore 110 may comprise a hybrid nanopore, in which a portal protein is assembled into a solid state nanopore 110 (e.g. as disclosed in Cressiot et al³ or any other suitable approach). The nanopore 110 may be formed with any suitable technique, such as by focussed ion beam or electron beam machining.
³ Cressiot, Benjamin, et al. "Thermostable virus portal proteins as reprogrammable adapters for solid-state nanopore sensors." Nature communications 9.1 (2018): 1-7.

The waveguide 105 may comprise a rib waveguide structure. For example, the waveguide 105 may comprise a silicon nitride (e.g. Si₃N₄) layer that has been partially etched to define a rib waveguide. The waveguide 105 may be configured to carry light with a wavelength in the infra-red or visible wavelength ranges (e.g. 380nm to 2000nm). In some embodiments the light may have a wavelength of less than 500nm. In some embodiments the waveguide may be configured to carry visible light (i.e. with a wavelength in the range 380nm to 700nm). The light may have a wavelength of 633-650nm, which may be compatible with certain fluorescent dies, and enable a relatively compact waveguide.

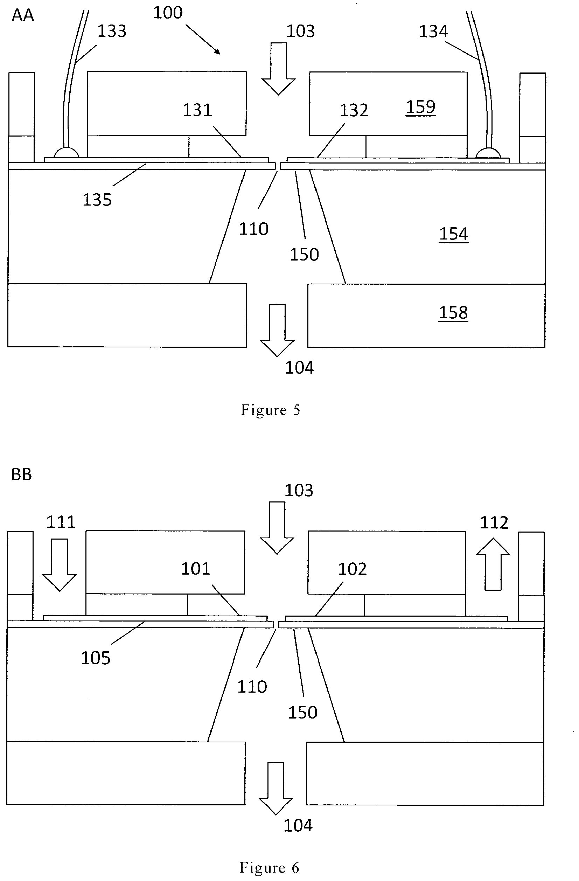

Figure 5 shows a schematic cross section along AA in Figure 1 (i.e. along the bowtie plasmonic enhancement structure 130). The nanopore 110 is defined in membrane 150, which may comprise a silicon nitride layer (for example). The membrane 150 is supported by a substrate 154 (e.g. comprising silicon), which has an opening that defines the membrane (e.g. formed by wet etching). The plasmonic enhancement structure 130 may be deposited on the membrane layer and patterned (e.g. lithographically). Where electrical contact to the plasmonic enhancement structure 130 is required, wire bonds 133, 134 may be used (or any other suitable approach, such as ball grid arrays, solder bumps etc.).

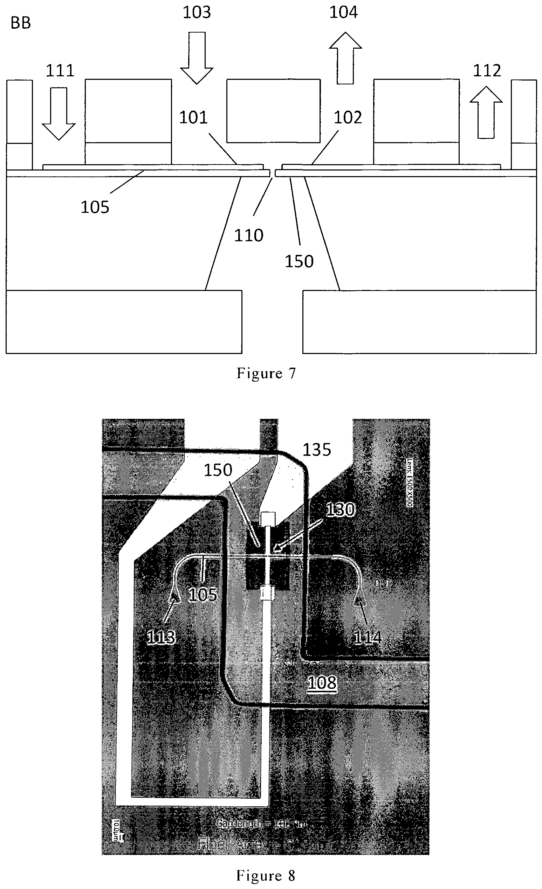

The nanophotonic nanopore 100 may be packaged to enable fluidic communication with the nanopore 110 (and potentially through the nanopore 110). In Figure 5 this is depicted schematically via top cap 159 and bottom cap 158, which provide a fluid input 103 and fluid output 104, but any suitable arrangement can be used (e.g. as in the alternative arrangements depicted schematically in Figures 4 and 8).

Figure 6 shows a schematic cross section along BB in Figure 1. The waveguide 105 is depicted as a separate layer to the membrane 150, but this is not essential, and the waveguide may be defined by a partial depth etch in the material comprising the membrane to define a rib waveguide structure. The first waveguide portion 101 is depicted as receiving light 111, for example via an out-of-plane grating coupler (not shown). The second waveguide portion 102 is depicted as outputting light 112 (after transmission via the region comprising the nanopore 110), for example via an out-of-plane grating coupler (not shown).

Figure 6 shows a schematic similar to that of Figure 6, comprising a fluid input 103 and fluid output 104 on a first side of the membrane 150 (in the case, the top side, but similar access can be achieved via the bottom side). Fluidic access to the second side (e.g. bottom) of the membrane 150 may be provided in addition to a cross flow fluidic communication with the first side of the membrane 150, as depicted in Figure 6.

In any of the embodiments, an analyte (e.g. DNA, or another polymer) may be received in the nanopore 110. In some embodiments, the analyte may be driven electrophoretically to be captured by and subsequently driven through the nanopore 110 (e.g. by the application of an electrical field across the thickness of the membrane).

Figure 8 shows a micrograph of a fabricated example device, based on the type of design shown in Figure 1. The membrane 150 is defined by an opening in the substrate. The waveguide 105 comprises a silicon nitride rib waveguide, and out-of-plane grating couplers 113, 114 are provided to communicate light to and from the waveguide 105. The material 135 comprising the plasmonic enhancement structure 130 is gold (with thickness ∼ 75nm). A fluidic channel 108 is defined in a top cap (similar to that shown in Figure 7). In this example the nanogap 142 was 100nm.

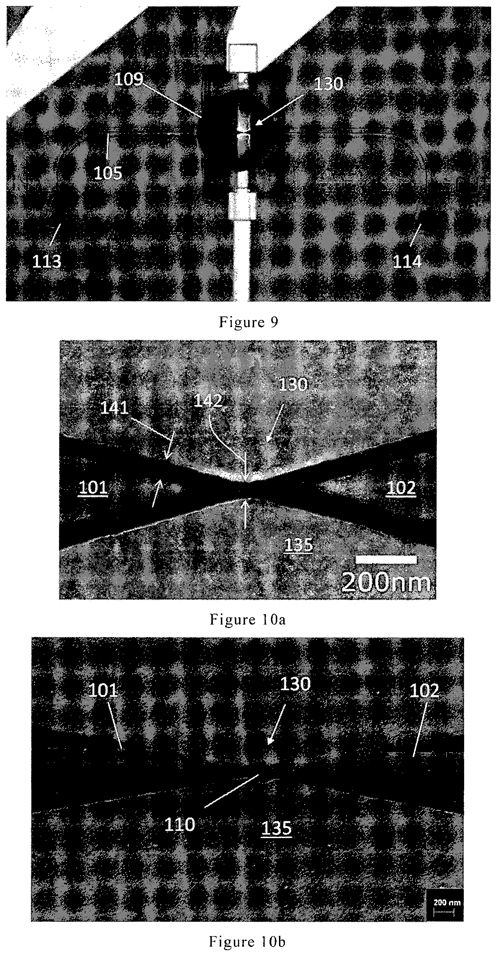

Figure 9 shows a micrograph of a fabricated example device, again based on the type of design shown in Figure 1, with a bowtie plasmonic enhancement structure. The fluidic channel 108 in this example is omitted, and instead a droplet 109 has been placed directly onto the region of the nanopore 110.

Figure 10 shows a micrograph close-up view of a bowtie type plasmonic enhancement structure 130, illustrating a nanogap 142 of approximately 50nm, and a taper gap of approximately 65nm. The nanopore 110 has not yet been formed.

Testing with devices according to embodiments (similar to those shown in Figures 5 to 8) show that nano particles (with sizes in the order of magnitude of atto-litres) are successfully captured by the nanopore 110 and that the presence of such particles can readily be detected by their effects on the transmission (reflection or absorption) properties of the waveguide 105.

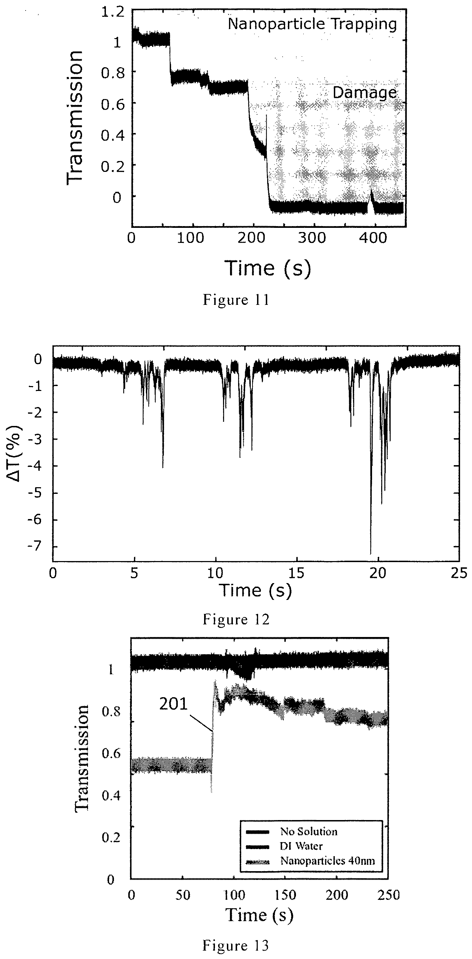

Figure 11 shows example results illustrating capture/trapping of nanoparticles at the nanopore 110, and the resulting changes in transmission of light through the waveguide 105. The particles here comprise 40nm gold nanoparticles with citrate stablisation. Testing of embdiments has demonstrate that reversible trapping of analytes in the nanopore is possible. A driving force (e.g. electrophoretic) may be used to trap the analyte, with the analyte becoming free again (and dispersed in the liquid again) on removal of the driving force (e.g. bias voltage).

Figure 12 shows results obtained from flowing a solution of nanoparticles over a nanopore (e.g. in the sort of flow arrangement shown in Figure 7), showing percentage changes in transmission as particles are captured by the nanopore.

Figure 13 shows normalised values of optical transmission through the waveguide with no fluid in contact with the nanopore 110, with deionised water in contact with the nanopore, and with a dispersion of 40nm particles in water in fluid communication with the nanopore. An increase in transmission around t=75s is associated with a particle detaching from the nanopore 110.

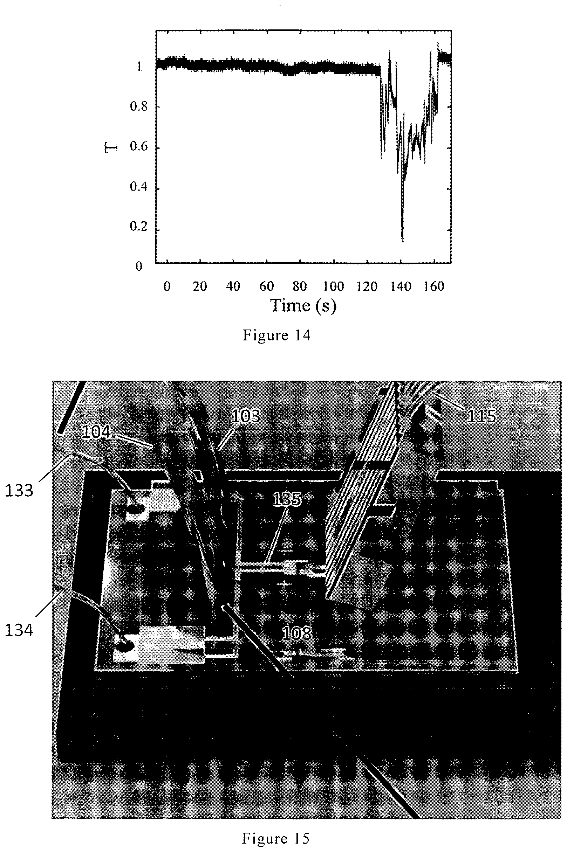

Figure 14 shows normalised values of transmission through a waveguide as fluid transits through the nanopore, showing marked drops in transmission as nanoparticles transit the nanopore.

Figure 15 shows a rendering of an example embodiment having fluid input 103 and fluid output 104 pipes, and wire bonds 133, 134 for addressing the electrodes of a bowtie plasmonic enhancement structure 130. Optical fibres 115 are coupled to out-of-plane grating couplers of the waveguide (not visible in Figure 15) to facilitate optical readout and/or writing of analytes or nanoparticles using the nanopore.

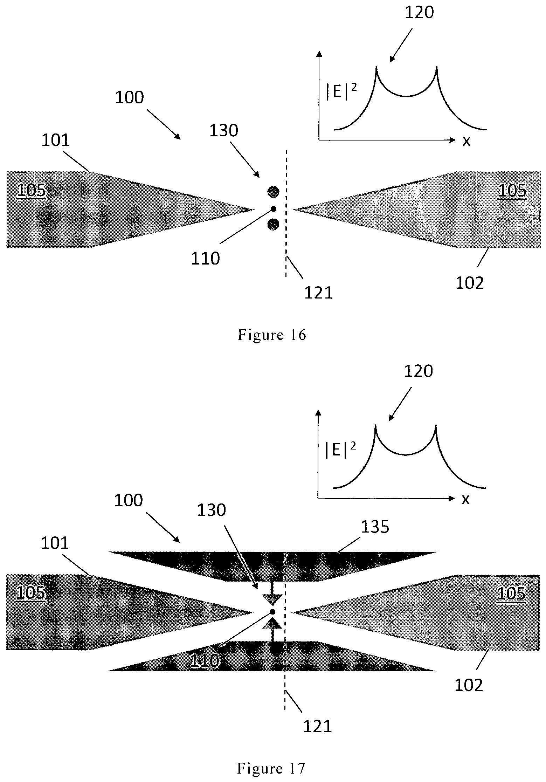

Although the foregoing examples have discussed a bowtie type plasmonic enhancement structure, other types of field enhancing structures can be used to enable optical interrogation of a nanopore. Figure 16 illustrates an example in which the waveguide 105 comprises a first waveguide portion 101 and a second waveguide portion 102 that each comprise male tapers pointing towards the nanopore 110. The plasmonic enhancement structure comprises a pair of plasmonic nano-discs. The plasmonic nano-discs are placed on either side of the nanopore on an axis perpendicular to the direction of light propagating through the waveguide 105. The field enhancement from this type of plasmonic enhancement structure is likely to be similar to that obtained with a bowtie structure. Discs, bowties and other structures that are not quasi-infinite will have a response that is strongly waveglength dependent. These resonances can be exploited to increase sensitivity to changes in the analyte within the nanpore. For example if the read wavelength is tuned to a resonance, it can be expected that the read signal will be sensitive to changes in the refractive index of the analyte (which may change the resonant properties of the plasmonic enhancement structure). The write wavelength may be different from the read wavelength, and not tuned to a resonance.

Figure 17 shows an example embodiment in which the plasmonic enhancement structure 130 comprises a plasmonic antenna. A plasmonic antenna similarly may have resonant properties that can be tuned to increase sensitivity in transduction (as outlined above).

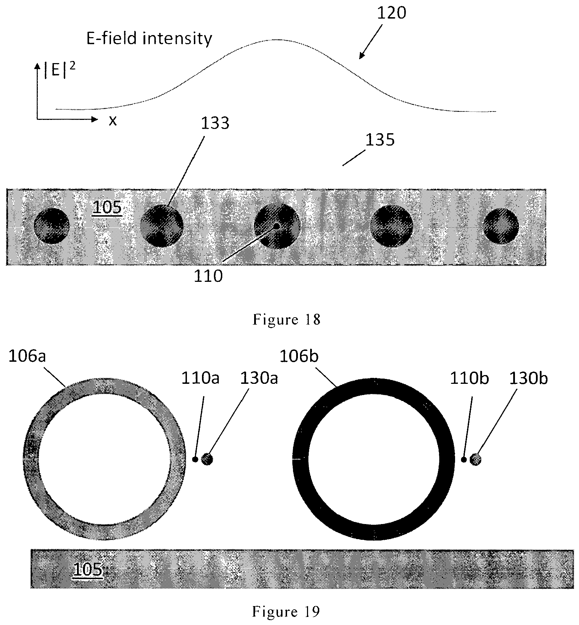

Figure 18 shows an example embodiment in which the field resulting from light propagating in the waveguide 105 is localised and enhanced by a photonic crystal defined in the waveguide 105, rather than by plasmonic enhancement. The photonic crystal comprises a series of cavities with sizes that vary with position, relative to the nanopore 110. The nanopore 110 is within the central, largest cavity (with cavities 133 decreasing in size with distance from the nanopore 110). The inset graph 120 shows a schematic illustration of the electric field intensity with respect to the position (x) along the waveguide 105.

Figure 19 illustrates a waveguide 105 coupled to more than one nanopore 110a, 110b, in this case via ring resonators 106a, 106b. The first ring resonator 106a is tuned to a first wavelength, and couples optical signals with the first wavelength from the waveguide 105 to interact with a first nanopore 110a (which is adjacent the first ring resonator 106a). A first plasmonic enhancement structure 130a adjacent the first nanopore 110a enhances and localises the field in the first nanopore 110a resulting from light propagating in the first ring resonator 106a. The second ring resonator 106b is tuned to a second wavelength (different from the first wavelength) and couples optical signals with the second wavelength to interact with a second nanopore 110b (which is adjacent the second ring resonator 106b). A second plasmonic enhancement structure 130b adjacent the second nanopore 110b enhances and localises the field in the second nanopore 110b resulting from light propagating in the second ring resonator 106b.

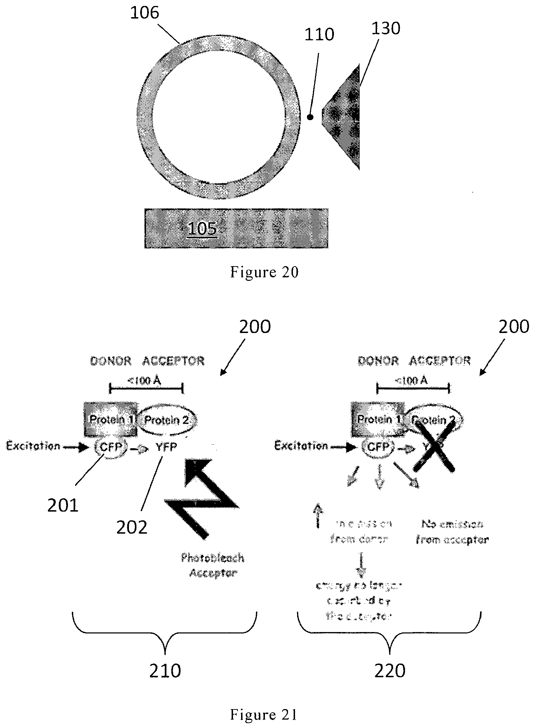

The plasmonic enhancement structures 130a, 130b in Figure 19 are nano-discs, but a half bowtie plasmonic enhancement structure can alternatively be used, as shown in Figure 20 (which shows only one ring resonator and nanopore, for simplicity).

In certain embodiments, a memory system is provided that uses the nanophotonic nanopore disclosed herein to read and/or write information from an analyte (e.g. information storing polymer). An information storing polymer can provide very high storage density. For example, it has been estimated⁴ that DNA has a storage density of 215 petabytes per gram. In some embodiments, a memory system may be provided that employs a nanophotonic nanopore to read base pairs of DNA.
⁴ Erlich, Yaniv, and Dina Zielinski. "DNA Fountain enables a robust and efficient storage architecture." Science 355.6328 (2017): 950-954.

It may be preferable to use a different informational polymer than DNA. A rationally designed informational polymer may be specifically configured to facilitate straightforward reading and writing by a nanophotonic nanopore according to an embodiment. Furthermore, an informational polymer may include position markers, for example to denote where data is written in the polymer. This may assist in accounting for variations in translocation rate of the polymer through the nanopore, and may enable different ends of the informational polymer to be distinguished (by marking the different ends with different markers). A rationally designed informational polymer may comprise encoding regions that are larger than those in DNA, so that they are easier to read as they pass through the nanopore. If the polymer can be written by a nanopore (e.g. optically) it becomes possible to easily synthesise a polymer and then inexpensively write information to it in bulk. Embodiments of the present nanophotonic nanopore can be used to read and/or write information to such an informational polymer.

Figure 21 provides a schematic illustration of an encoding unit 200 of a suitable informational polymer. The encoding unit 200 comprises a first fluorescent protein 201 and a second fluorescent protein 202 that exhibit fluorescence resonance energy transfer (FRET). The first fluorescent protein 201 may be a cyan fluorescent protein (CFP), and the second fluorescent protein 202 may be a yellow fluorescent protein (YFP).

In an initial state 210 (which may, for example, represent the symbol '0'), excitation of the first fluorescent protein 201 results in energy transfer to the second fluorescent protein 202, with the result that the spectrum emitted from the encoding unit 200 has a first ratio of cyan to yellow (e.g. dominated by yellow fluorescent emission). Exposure of the encoding unit 200 to light (with a predetermined spectra and intensity) may result in bleaching of the second fluorescent protein 202 (or a disruption of the FRET mechanism), with the result that excitation of the first fluorescent protein 201 no longer results in energy transfer and fluorescence of the second fluorescent protein 202. The result of the photobleaching is therefore that the fluorescent spectrum from the encoding unit 200 is changed to comprise more fluorescence from the first fluorescent protein 201 than the second fluorescent protein 202. The photobleached state 220 can encode the symbol '1', for example. In some embodiments, the degree of photobleaching may be used to encode more than one symbol (e.g. based on a ratio of cyan to yellow fluorescence).

In some embodiments, light carried by the waveguide may be used to impart a structural change in the informational polymer. A polymerisation or other reaction between two compounds comprising the encoding unit may be used to change an excitation emission, or Raman spectrum, from the encoding unit.

In some embodiments, the nanophotonic nanopore may be used to determine a Raman spectrum of the region of the analyte (e.g. informational polymer) captured by the nanopore. The Raman spectrum may provide a relatively precise chemical footprint of the encoding region of the analyte, thereby enabling reading of the information content encoded by the encoding region.

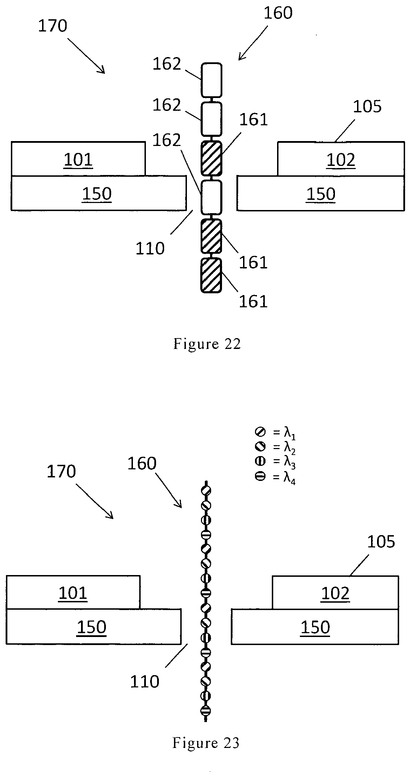

Figure 22 schematically illustrates a memory system 170, comprising an analyte 160 and nanophotonic nanopore comprising waveguide 105, membrane 150 and nanopore 110. The analyte 160 is an informational polymer, which is captured by the nanopore 110. As the analyte 160 translates through the nanopore 110, each successive encoding region can be read and/or written using light guided by the waveguide 105. In this example, encoding regions 161 encode symbol "1" and encoding regions 162 encode symbol "0". In other embodiments systems with more than two symbols may be employed, so that each encoding region may store more than 1 bit of data.

Figure 23 schematically illustrates a memory system 170, comprising an analyte 160 and nanophotonic nanopore comprising waveguide 105, membrane 150 and nanopore 110. The analyte 160 is an informational polymer, which is captured by the nanopore 110. As the analyte 160 translates through the nanopore 110, successive encoding regions can be read and/or written using light guided by the waveguide 105. In this example, encoding regions are provided with subregions that can be read/written using light with distinct wavelengths. In this example each encoding region comprises four sub-regions, each read and/or written using a different wavelength of light. All four sub-regions in each encoding region may thereby be simultaneously read and/or written via the waveguide 105 simultaneously, using a form of wavelength division multiplexing. This is one way of storing more than one bit of data within each encoding region (and subsequently reading more than one bit of information in parallel from one waveguide).

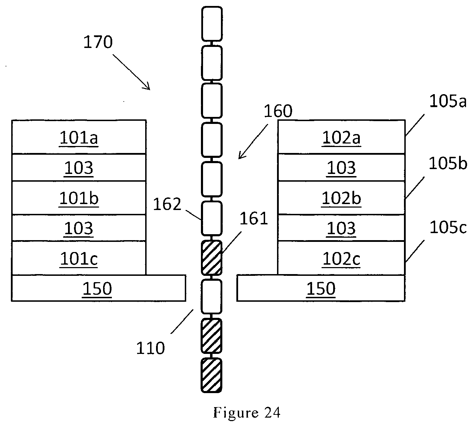

Figure 24 schematically illustrates a memory system 170, comprising an analyte 160 and nanophotonic nanopore comprising waveguides 105a-c, membrane 150 and nanopore 110. The analyte 160 is an informational polymer, which is captured by the nanopore 110. In this example there are a plurality of stacked waveguides 105a-c, which are spaced apart. In some embodiments the stacked waveguides 105a-c may be used to enable parallel read and/or write operations to be performed at more than one encoding region of the analyte 160. In other embodiments, different waveguides 105a-c may be used to perform different tasks. For example waveguide 105a may be used to check the translocation rate of the analyte through the nanopore. Waveguide 105b may be used to read and/or write to information encoding regions of the analyte as they pass waveguide 105b. Waveguide 105c may be used to check the result of the read and/or write operation performed at waveguide 105b. In the case of a write operation, waveguides 105b and 105c can be thought of as a "write" waveguide and a "verify" waveguide, which reads the encoded information to check that it is correctly recorded.

Although it may not be possible to fix write errors, keeping a record of the number of write errors per encoding unit in a batch of informational polymers will enable an estimate of the number of read operations required per encoding location to get a reliable read of the desired data.

Stochastic sampling of information encoded by polymers may be used to retrieve data, even when there are write errors. In addition, information may be encoded using error correcting schemes that allow for the expected error rate associated with writing and reading the information via the informational polymer.

In some embodiments, specific informational polymers may comprise a magnetic nanoparticle, which may enable selection of different strands of informational polymer, thereby enabling some form of memory addressing (beyond random sampling of informational polymers). In some embodiments nanopores of different sizes may be configured to each capture only a specific range of analytes. In some embodiments, the analyte may be captured and/or driven through the nanopore by a combination of dielectrophoresis and electrophoresis.

Although specific examples and embodiments have been described, the scope of the invention is to be determined with reference to the appended claims.

## Claims

1. A nanophotonic nanopore comprising:
a nanopore, and an optical waveguide;
wherein the optical waveguide is optically coupled to the nanopore so that a transmission, reflection, emission or absorption property of the optical waveguide is modified by an analyte received by the nanopore.

2. The nanophotonic nanopore of claim 1, further comprising a plasmonic enhancement structure configured to enhance an electrical field strength at the nanopore by field confinement of an optical signal carried by the optical waveguide.

3. The nanophotonic nanopore of claim 2, wherein the plasmonic enhancement structure comprises a first region and second region, spaced apart by a nanogap at the nanopore.

4. The nanophotonic nanopore of claim 3, including the subject matter of claim 2, wherein:
the optical waveguide comprises a first waveguide portion and a second waveguide portion that are coupled together via the nanopore;
the first waveguide portion comprises a narrowing taper at an end of the first waveguide portion adjacent to the nanopore,
the second waveguide portion comprises a narrowing taper at an end of the second waveguide portion adjacent to the nanopore,
the plasmonic enhancement structure is spaced apart from the narrowing tapers of the first and second waveguide portions by a taper nanogap.

5. The nanophotonic nanopore of any of claims 2 to 4, wherein the plasmonic enhancement structure comprises: silver, gold, aluminium, copper, titanium nitride, or a heavily doped semiconductor material.

6. The nanophotonic nanopore of any preceding claim, further comprising a first electrode and a second electrode that are separated by a plasmonic nanogap at the nanopore.

7. The nanophotonic nanopore of claim 1 or 6, wherein the optical waveguide is configured as a photonic crystal comprising at least one cavity, and the nanopore is disposed within one of the at least one cavities, and the photonic crystal is optionally configured to enhance the electric field in the region of the nanopore resulting from an optical signal propagating through the optical waveguide.

8. The nanophotonic nanopore of any preceding claim, wherein the optical waveguide comprises one of a plurality of optical waveguides in a stacked configuration, each of the optical waveguides optically coupled to the nanopore so that a transmission property of each optical waveguide is modified by a respective region of the analyte at the position of that optical waveguide.

9. The nanophotonic nanopore of any preceding claim, wherein the waveguide is configured to carry light with a wavelength that is at least twice a diameter of the nanopore.

10. A memory system, comprising:
a nanophotonic nanopore according to any preceding claim, and
an analyte comprising one or more information encoding regions that are configured to change a characteristic in response to an optical signal carried by the optical waveguide when the information encoding region is within the nanopore;
wherein the nanophotonic nanopore is configured to write information to the analyte by changing the characteristic of the information encoding region.

11. A memory system, comprising:
a nanophotonic nanopore according to any of claim 1 to 9, and
an analyte comprising one or more information encoding regions that comprise a characteristic that can be inferred by transmission properties of the optical waveguide when the information encoding region is within the nanopore;
wherein the nanophotonic nanopore is configured to read information encoded in the characteristic of the information encoding regions of the analyte.

12. The memory system of any of claims 10 or 11, wherein the encoding region comprises a fluorophore.

13. The memory system of claim 12, wherein the encoding region comprises a first fluorophore and a second fluorophore, and the first and second fluorophore are coupled by fluorescence resonance energy transfer, so that excitation of the first fluorophore results in emission from the second fluorophore, and wherein the memory system is optionally configured so that writing to the encoding region comprises bleaching the encoding region to reduce a fluorescence resonance energy transfer.

14. The memory system of any of claims 10 to 13, wherein the nanophotonic nanopore is configured to read multiple bits of information in parallel using different wavelengths.

15. A method of reading and/or writing information from an analyte, comprising:
capturing the analyte in a nanophotonic nanopore, the nanophotonic nanopore comprising a nanopore and an optical waveguide optically coupled to the nanopore;
optically reading and/or writing information to the analyte using an optical signal carried by the waveguide.
